# EUROPEAN PATENT APPLICATION

(11) **EP 4 331 719 A1**
(43) Date of publication of application: **06.03.2024**
(21) Application number: 22815935.6
(22) Date of filing: 25.05.2022
(51) Int. Cl.: B01J 19/12

(54) **ULTRAVIOLET RAY RADIATION APPARATUS**

(30) Priority: 03.06.2021 JP 2021093634
(71) Applicant: DAIKIN INDUSTRIES, LTD., Osaka-Shi, Osaka 530-0001 (JP)
(72) Inventor: SAITO, Tomoki, Osaka-shi, Osaka 530-0001 (JP); KUROI, Kiyoshi, Osaka-shi, Osaka 530-0001 (JP); TANAKA, Toshio, Osaka-shi, Osaka 530-0001 (JP); KOYAMA, Chika, Osaka-shi, Osaka 530-0001 (JP); OKUMOTO, Mamoru, Osaka-shi, Osaka 530-0001 (JP)
(74) Representative: Hoffmann Eitle
(86) International application number: PCT/JP2022/021396
(87) International publication number: WO 2022/255185

(57) **Abstract**

An ultraviolet ray radiation apparatus (100) includes a light emitter (101) emitting ultraviolet light, and a light receiver (102) outputting a signal varying with an amount of light received. The ultraviolet ray radiation apparatus (100) further includes a protector (104A, 104B) protecting the light receiver (102) from ultraviolet light.

## Description

### TECHNICAL FIELD

The present disclosure relates to an ultraviolet ray radiation apparatus.

### BACKGROUND ART

An ultraviolet ray radiation apparatus has been utilized to remove viruses, bacteria, mold, or the like. Examples of a light source of the ultraviolet ray radiation apparatus include a lamp, a light emitting diode (LED), and the like. These light sources vary in the amount of light, e.g., they emit a less amount (intensity) of light due to the influence of an ambient temperature or with the passage of time of the use thereof.

Patent Document 1 discloses detecting the intensity of light emitted from an LED to control the LED, thereby keeping the amount of light emitted constant.

### CITATION LIST

### PATENT DOCUMENTS

Patent Document 1: Japanese Unexamined Patent Publication No. 2010-275840

### SUMMARY OF THE INVENTION

### TECHNICAL PROBLEM

However, a photodetector of the apparatus disclosed in Patent Document 1 deteriorates due to ultraviolet light with high energy intensity. The deterioration of the photodetector makes it difficult to stably control the LED.

It is an object of the present disclosure to provide an ultraviolet ray radiation apparatus that can stably control a light emitter that emits ultraviolet light.

### SOLUTION TO THE PROBLEM

A first aspect of the present disclosure is directed to an ultraviolet ray radiation apparatus including: a light emitter (101) configured to emit ultraviolet light; a light receiver (102) configured to output a signal varying with an amount of light received; and a protector (104A, 104B, 104C) configured to protect the light receiver (102) from ultraviolet light.

According to the first aspect, the light receiver (102) outputting a signal varying with the amount of light received is protected from ultraviolet light by the protector (104A, 104B, 104C). This can reduce deterioration of the light receiver (102) with the passage of time of the use thereof. Thus, the amount of ultraviolet light emitted from the light emitter (101) can be stably controlled based on the signal from the light receiver (102).

A second aspect of the present disclosure is an embodiment of the first aspect. In the second aspect, the protector (104A, 104B, 104C) attenuates an amount of ultraviolet light entering the light receiver (102).

According to the second aspect, the ultraviolet light emitted from the light emitter (101) enters the light receiver (102) via the protector (104A, 104B, 104C), and this leads to a reduction in the amount of irradiation of the light receiver (102) with ultraviolet light with high energy intensity and great damage power.

A third aspect of the present disclosure is an embodiment of the first or second aspect. In the third aspect, the protector (104A, 104B, 104C) includes a reflector (104B) configured to reflect ultraviolet light at least once until the ultraviolet light enters the light receiver (102) from the light emitter (101).

According to the third aspect, light with a short wavelength such as ultraviolet light is more easily attenuated by reflection than light with a long wavelength. Thus, ultraviolet light emitted from the light emitter (101) is reflected once or more by the reflector (104B) and then enters the light receiver (102), such that the light receiver (102) can be less deteriorated.

A fourth aspect of the present disclosure is an embodiment of any one of the first to third aspects. In the fourth aspect, the protector (104A, 104B, 104C) includes a light shield (104C) configured to block light with a wavelength shorter than a peak wavelength of ultraviolet light emitted from the light emitter (101).

According to the fourth aspect, the ultraviolet light emitted from the light emitter (101), which particularly has a shorter wavelength to provide great damage power, is blocked. Thus, the light receiver (102) can be less deteriorated.

A fifth aspect of the present disclosure is an embodiment of any one of the first to fourth aspects. In the fifth aspect, the protector (104A, 104B, 104C) includes a light shield (104C) configured to block light with a wavelength less than or equal to 280 nm.

According to the fifth aspect, the ultraviolet light emitted from the light emitter (101), which particularly has a wavelength less than or equal to 280 nm to provide great damage power, is blocked. Thus, the light receiver (102) can be less deteriorated.

A sixth aspect of the present disclosure is an embodiment of any one of the first to fifth aspects. In the sixth aspect, the light emitter (101) is an LED (101).

According to the sixth aspect, the light emitter (101) can achieve downsizing, weight-reduction, less power consumption, and the like.

A seventh aspect of the present disclosure is an embodiment of the sixth aspect. In the seventh aspect, the ultraviolet ray irradiation apparatus further includes a control unit (103) configured to adjust a driving current of the LED (101) based on a signal from the light receiver (102) to control an amount of light emitted from the LED (101).

According to the seventh aspect, the driving current of the LED (101) is adjusted so that the signal from the light receiver (102) (i.e., the amount of light received) is constant. Thus, the amount of light emitted from the LED (101) can be made constant.

An eighth aspect of the present disclosure is an embodiment of any one of the first to seventh aspects. In the eighth aspect, an optical filter (105) configured to block light with a wavelength greater than or equal to 300 nm is provided in front of the light receiver (102).

According to the eighth aspect, wavelength band components (disturbance components) of ultraviolet rays emitted from the light emitter (101), where the wavelength band components are contained in sunlight and typical illumination light, can be blocked by the optical filter (105). Thus, the amount of light emitted from the light emitter (101) can be accurately controlled based on the signal from the light receiver (102).

A ninth aspect of the present disclosure is an embodiment of any one of the first to eighth aspects. In the ninth aspect, the light emitter (101) and the light receiver (102) are integrated together.

According to the ninth aspect, the ultraviolet ray radiation apparatus (100) can be downsized. In addition, it is easier to determine the positional relationship between the light emitter (101) and the light receiver (102), the specifications of peripheral members, and the like. Thus, desired functions can be more reliably achieved.

### BRIEF DESCRIPTION OF THE DRAWINGS

FIG. 1 is a vertical sectional view of an indoor unit of an air conditioner including an ultraviolet ray radiation apparatus according to an embodiment.
FIG. 2 is a schematic view illustrating an exemplary configuration of an ultraviolet ray radiation apparatus according to an embodiment.
FIG. 3 is a schematic view illustrating an exemplary configuration of an ultraviolet ray radiation apparatus according to an embodiment.
FIG. 4 is a schematic view illustrating an exemplary configuration of an ultraviolet ray radiation apparatus according to an embodiment.
FIG. 5 is a block diagram illustrating an exemplary configuration of a control unit of an ultraviolet ray radiation apparatus according to an embodiment.
FIG. 6 is a schematic view illustrating an exemplary configuration of an ultraviolet ray radiation apparatus according to an embodiment.
FIG. 7 is a schematic view illustrating an exemplary configuration of an ultraviolet ray radiation apparatus according to an embodiment.
FIG. 8 is a see-through perspective view of an air cleaner including an ultraviolet ray radiation apparatus according to an embodiment.
FIG. 9 is a schematic view illustrating a configuration of an essential portion of the air cleaner illustrated in FIG. 8.

### DESCRIPTION OF EMBODIMENTS

### (Embodiment)

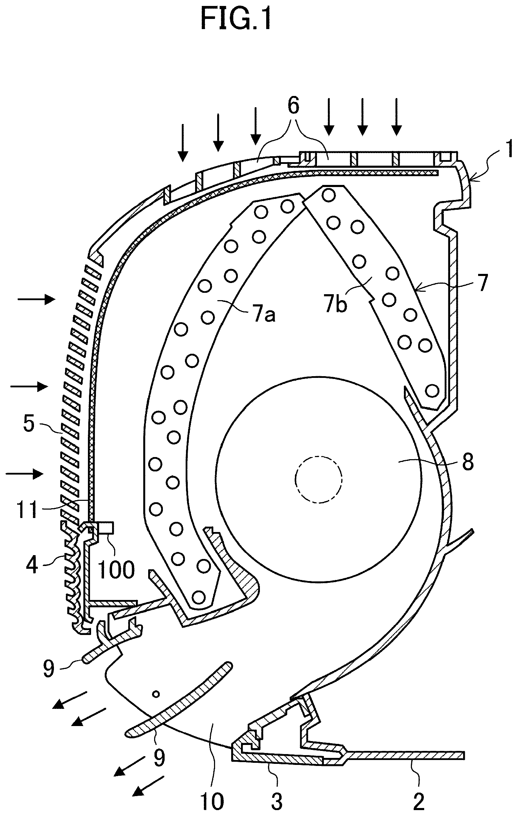

An embodiment will be described with reference to the drawings. An ultraviolet ray radiation apparatus (100) of this embodiment is provided in, e.g., an indoor unit of an air conditioner illustrated in FIG. 1. The indoor unit includes a casing (1) with a generally rectangular cross section. The casing (1) includes a bottom frame (2) fixed along an indoor wall surface and located near its back surface (on the right side of FIG. 1), and a front grille (3) attached to the front surface of the bottom frame (2). A front panel (4) is attached to the front grille (3) to cover an opening of the front surface of the front grille (3), and the front panel (4) has front inlets (5). A top panel surface of the casing (1) has upper inlets (6). The casing (1) accommodates a heat exchanger (7) and a fan (8). The heat exchanger (7) functions as a condenser (during a heating operation) and an evaporator (during a cooling operation) in a refrigerant circuit. The fan (8) is configured as a cross-flow fan. The heat exchanger (7) includes a front heat exchanger (7a) and a back heat exchanger (7b) combined together in the form of an inverted V shape. The fan (8) is arranged between lower end portions of the front heat exchanger (7a) and the back heat exchanger (7b). The front grille (3) includes a lower front portion having an outlet (10) provided with one or more horizontal flaps (9) and one or more vertical flaps (not shown). A dust filter (11) is fitted into the casing (1) along the inner sides of the front inlets (5) and the upper inlets (6).

In the indoor unit illustrated in FIG. 1, the ultraviolet ray radiation apparatus (100) is installed in a lower space between the dust filter (11) and the front heat exchanger (7a). The ultraviolet ray radiation apparatus (100) may be supported by a holder (not shown). The ultraviolet ray radiation apparatus (100) is configured to be capable of irradiating nearly the entire surface of the dust filter (11) with ultraviolet light. Irradiation with ultraviolet light enables sterilization of the dust filter (11).

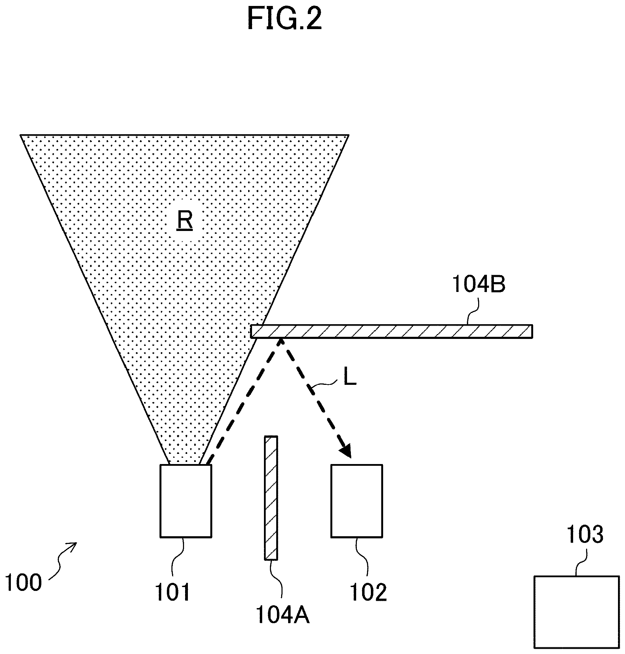

As illustrated in FIG. 2, the ultraviolet ray radiation apparatus (100) mainly includes a light emitter (101) emitting ultraviolet light, a light receiver (102) outputting a signal varying with the amount of light received, and protectors (104A, 104B) protecting the light receiver (102) from ultraviolet light. The ultraviolet ray radiation apparatus (100) may further include a control unit (103) controlling the light emitter (101) based on the signal from the light receiver (102). Alternatively, the control unit (103) may be configured as a part of a control unit of the air conditioner or the indoor unit. In FIG. 2, a region irradiated with ultraviolet light (at an intensity greater than or equal to a predetermined intensity) by the ultraviolet ray radiation apparatus (100) is indicated by "R," and a path through which ultraviolet light emitted from the light emitter (101) enters the light receiver (102) is indicated by "L."

The emission wavelength of the light emitter (101) may be, e.g., less than or equal to about 380 nm, or may be, e.g., less than or equal to about 300 nm to provide a strong bactericidal effect. The light emitter (101) may be, e.g., one or more LEDs such as a semiconductor light emitting element including a light emitting layer made of a nitride semiconductor, or may be, e.g., a mercury lamp, an excimer lamp, or the like. If one or more LEDs are used, the dimension of the LED, the number of LEDs installed, the installation distance between LEDs, and the like can be appropriately set in accordance with a target to be irradiated with ultraviolet light. If a plurality of LEDs are used, the LEDs may all be of the same type, or the LEDs may have different emission wavelengths or different optical output powers. The LED may be a light emitting element contained in a package, or may be a bare-chip light emitting element.

The light receiver (102) outputs a signal (a current value or a voltage value) varying with the amount of light received (the energy intensity of incident light), at the sensitivity determined depending on the light wavelength. The light receiver (102) is any light receiver having sensitivity to ultraviolet light emitted from the light emitter (101), and may be a light receiving element made of a semiconductor material or the like, such as a photodiode, a phototransistor, a photocapacitor, or the like, or may be a photomultiplier (a photomultiplier tube). The light receiver (102) may be a light receiving element or the like contained in a package, or may be a bare-chip light receiving element or the like.

The control unit (103) controls the amount of light emitted from the light emitter (101), specifically, the emission intensity or the emission time, based on the signal output from the light receiver (102). The amount of light emitted (dose (J/cm²)) is equal to the product of the emission intensity (irradiation intensity (W/cm²)) and the emission time (irradiation time (second)). If the light emitter (101) is one or more LEDs, the control unit (103) controls the driving current of the one or more LEDs to control the amount of light emitted from the one or more LEDs. If the light emitter (101) is one or more lamps, the control unit (103) controls the number of lamps activated or the switching-on/off of the lamp(s) to control the amount of light emitted from the one or more lamps. The control unit (103) may be configured to include, e.g., a microcomputer (not shown) and a program for operating the microcomputer. The control unit (103) may be configured as a part of the control unit of the indoor unit illustrated in FIG. 1.

In the configuration illustrated in FIG. 2, the protectors (104A, 104B) include a light shield (104A) preventing ultraviolet light from entering the light receiver (102) directly from the light emitter (101), and a reflector (104B) reflecting ultraviolet light emitted from the light emitter (101) toward the light receiver (102). The light shield (104A) and the reflector (104B) may attenuate the amount of ultraviolet light entering the light receiver (102). The material of each of the light shield (104A) and the reflector (104B) may be any material that absorbs or reflects ultraviolet light. The material may be, e.g., fiber-reinforced plastics, zinc oxide, titanium oxide, or the like. The light shield (104A) may be arranged between the light emitter (101) and the light receiver (102) to hide the light emitter (101) when viewed from the light receiver (102). The reflector (104B) may be arranged apart from the light emitter (101) and the light receiver (102). The reflector (104B) may be arranged to reflect, toward the light receiver (102), relatively-low-intensity light (e.g., light present outside the irradiation region R) out of ultraviolet light emitted from the light emitter (101). The reflector (104B) may include a plurality of reflectors (104B) so that ultraviolet light is reflected twice or more before the ultraviolet light enters the light receiver (102) from the light emitter (101).

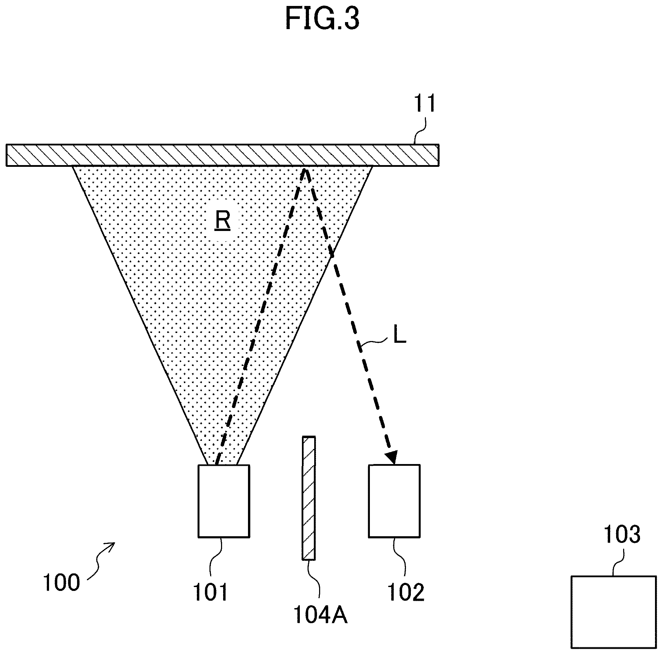

If a target to be irradiated with ultraviolet light by the ultraviolet ray radiation apparatus (100) is made of a material that reflects ultraviolet light, the ultraviolet ray radiation apparatus (100) may be provided without the reflector (104B) so that ultraviolet light having reflected off the target (e.g., the dust filter (11)) to be irradiated with ultraviolet light enters the light receiver (102) as illustrated in FIG. 3. In FIG. 3, the same reference characters are used to designate the same elements as those of the ultraviolet ray radiation apparatus (100) illustrated in FIG. 2.

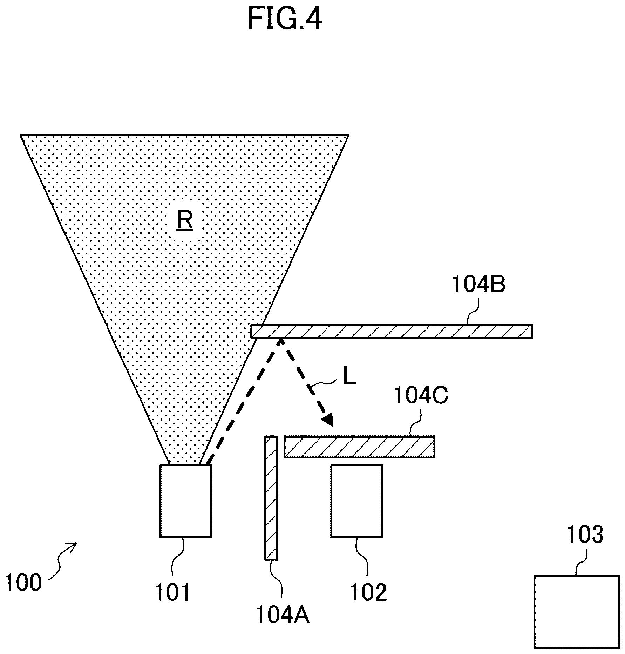

If the reflector (104B) does not attenuate a sufficient amount of ultraviolet light, the ultraviolet ray radiation apparatus (100) may further include a light shield (104C) as a protector for the light receiver (102) on the path L of ultraviolet light from the reflector (104B) to the light receiver (102), as illustrated in FIG. 4. In FIG. 4, the same reference characters are used to designate the same elements as those of the ultraviolet ray radiation apparatus (100) illustrated in FIG. 2. The light shield (104C) may selectively block light with a wavelength shorter than the peak wavelength of ultraviolet light emitted from the light emitter (101), for example. The light shield (104C) may selectively block light with a wavelength less than or equal to 280 nm, for example. The light shield (104C) may be configured as an optical element such as an optical filter, or may be made of a light attenuating material or the like. The light attenuating material may be, e.g., a thin plate of metal or resin with holes, a metal mesh, or the like.

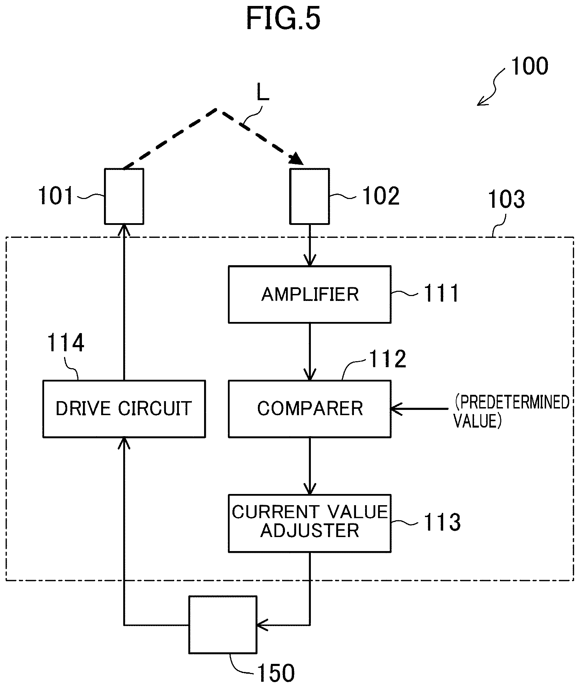

A detailed configuration example of the control unit (103) in a situation where the light emitter (101) is configured as an LED (which may be hereinafter referred to as the "LED (101)") will be described below. As shown in FIG. 5, the control unit (103) is electrically connected to a power source (150), and current from the power source (150) is supplied to the LED (101) through the control unit (103). The control unit (103) controls the amount of light emitted from the LED (101) by increasing or decreasing the amount of current during constant voltage driving of the LED (101), or by increasing or decreasing the emission time or the emission interval during pulse driving of the LED (101). Specifically, the control unit (103) includes an amplifier (111) amplifying the signal (e.g., current) output from the light receiver (102), a comparer (112) comparing the value of the amplified signal to a predetermined value, and a current value adjuster (113) adjusting a target current value of the power source (150) based on the output of the comparer (112). The control unit (103) further includes a drive circuit (114) driving and controlling light emissions from the LED (101). The current from the power source (150) adjusted by the current value adjuster (113) is supplied to the LED (101) through the drive circuit (114). The predetermined value used for the comparer (112) may be stored in a storage incorporated in the control unit (103). The predetermined value is appropriately set in accordance with the structure of the target to be irradiated with ultraviolet light, the configuration of the LED (101), the purpose of irradiation with ultraviolet light (e.g., sterilization), and the like.

The LED (101) emits a less amount of light at the same driving current with the passage of time of the use thereof. In other words, to maintain the same amount of light emitted, the LED (101) needs larger driving current with the passage of time of the use thereof. However, the LED (101) has an upper limit of the driving current value. Thus, when the target current value adjusted by the current value adjuster (113) reaches the upper limit of the driving current value of the LED (101), the control unit (103) may inform a user or the like through sound or light that the LED (101) has ended its life.

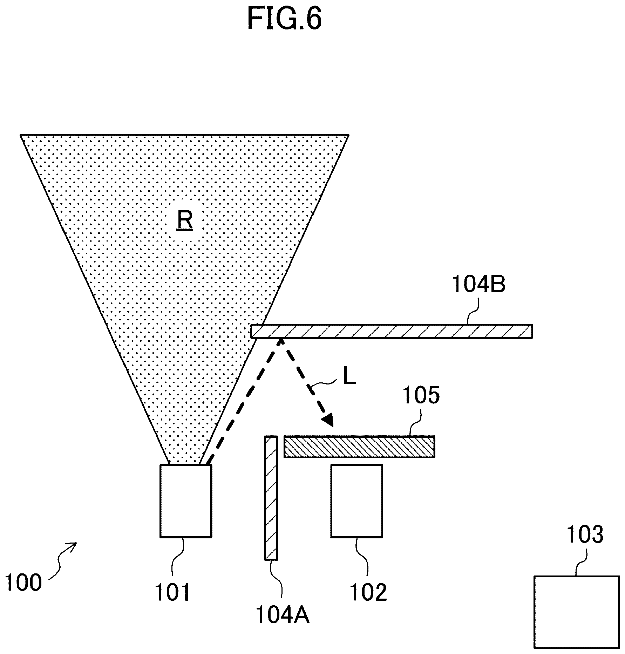

Sunlight reaching the ground and light emitted from interior lighting contain ultraviolet light with a wavelength greater than or equal to 300 nm. If this ultraviolet component serves as a disturbance and enters the light receiver (102), the amount of ultraviolet light emitted from the light emitter (101) cannot be detected accurately. Thus, as illustrated in FIG. 6, the ultraviolet ray radiation apparatus (100) may include an optical filter (105) provided in front of the light receiver (102) to block light with a wavelength greater than or equal to 300 nm. In FIG. 6, the same reference characters are used to designate the same elements as those of the ultraviolet ray radiation apparatus (100) illustrated in FIG. 2. The optical filter (105) is provided such that only ultraviolet light with a wavelength band that is not present in an ordinary space can enter the light receiver (102). Thus, the amount of ultraviolet light emitted only from the light emitter (101) can be detected accurately without being affected by the disturbance. The light shield (104C) illustrated in FIG. 4 may be arranged in front of the light receiver (102) together with the optical filter (105). Alternatively, a light shielding member functioning as both the light shield (104C) and the optical filter (105) may be arranged in front of the light receiver (102).

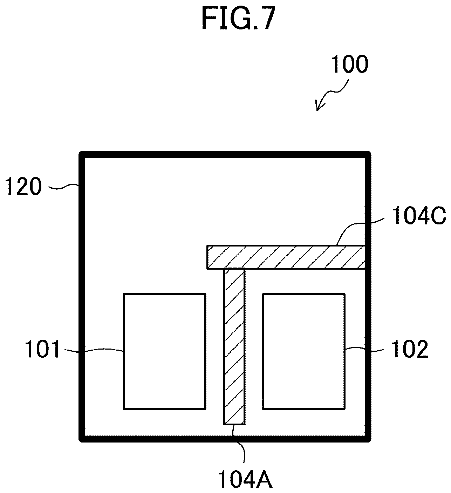

If a space where the ultraviolet ray radiation apparatus (100) is arranged is small just like the interior of the indoor unit illustrated in FIG. 1, the light emitter (101) and the light receiver (102) may be integrated together. For example, as illustrated in FIG. 7, the light emitter (101) and the light receiver (102) may be provided in the same package (120). In this case, the light shields (104A, 104C) described above may be provided as protectors for the light receiver (102) in the same package (120). The package (120) is configured so that ultraviolet light emitted from the light emitter (101) can reach a target (outside the drawing) to be irradiated and ultraviolet light having reflected off a reflector (outside the drawing) or the like can reach the light receiver (102). Instead of the light emitter (101) and the light receiver (102) being provided in the same package (120), the light emitter (101) and the light receiver (102) may be mounted on the same substrate.

### -Features of Embodiment-

As described above, the ultraviolet ray radiation apparatus (100) of this embodiment includes the light emitter (101) emitting ultraviolet light, the light receiver (102) outputting a signal varying with the amount of light received, and the protectors (104A, 104B, 104C) protecting the light receiver (102) from ultraviolet light.

According to the ultraviolet ray radiation apparatus (100) of this embodiment, the protectors (104A, 104B, 104C) protect, from ultraviolet light, the light receiver (102) outputting a signal varying with the amount of light received. Thus, the light receiver (102) is less deteriorated with the passage of time of the use thereof. Thus, the amount of ultraviolet light emitted from the light emitter (101) can be stably controlled based on the signal from the light receiver (102). In addition, the performance of the ultraviolet ray radiation apparatus (100) can be assured, whereas the substantial life of the ultraviolet ray radiation apparatus (100) can be made larger without overdesigning.

In the ultraviolet ray radiation apparatus (100) of this embodiment, the protectors (104A, 104B, 104C) may attenuate the amount of ultraviolet light entering the light receiver (102). According to this, ultraviolet light emitted from the light emitter (101) enters the light receiver (102) via the protectors (104A, 104B, 104C), and thus the light receiver (102) can be less irradiated with ultraviolet light having high energy intensity and great damage power.

In the ultraviolet ray radiation apparatus (100) of this embodiment, the protectors (104A, 104B, 104C) may include the reflector (104B) reflecting ultraviolet light at least once until the ultraviolet light enters the light receiver (102) from the light emitter (101). Light with a short wavelength such as ultraviolet light is more easily attenuated by reflection than light with a long wavelength. Thus, ultraviolet light emitted from the light emitter (101) is reflected once or more by the reflector (104B) and then enters the light receiver (102), such that the light receiver (102) can be less deteriorated.

In the ultraviolet ray radiation apparatus (100) of this embodiment, the protectors (104A, 104B, 104C) may include the light shield (104C) blocking light with a wavelength shorter than the peak wavelength of ultraviolet light emitted from the light emitter (101) or light with a wavelength less than or equal to 280 nm. According to this, the ultraviolet light emitted from the light emitter (101), which particularly has a shorter wavelength or a wavelength less than or equal to 280 nm to provide great damage power, is blocked. Thus, the light receiver (102) can be less deteriorated.

In the ultraviolet ray radiation apparatus (100) of this embodiment, the light emitter (101) may be an LED (101) that can achieve downsizing, weight-reduction, less power consumption, and the like. In this case, the control unit (103) may be further provided that adjusts the driving current of the LED (101) based on the signal from the light receiver (102) to control the amount of light emitted from the LED (101). In this case, the driving current of the LED (101) is adjusted so that the signal from the light receiver (102) (i.e., the amount of light received) is constant. Thus, the amount of light emitted from the LED (101) can be made constant.

In the ultraviolet ray radiation apparatus (100) of this embodiment, the optical filter (105) blocking light with a wavelength greater than or equal to 300 nm may be provided in front of the light receiver (102). According to this, wavelength band components (disturbance components) of ultraviolet rays emitted from the light emitter (101), where the wavelength band components are contained in sunlight, illumination light, and the like, can be blocked by the optical filter (105). Thus, the amount of light emitted from the light emitter (101) can be controlled accurately based on the signal from the light receiver (102).

In the ultraviolet ray radiation apparatus (100) of this embodiment, the light emitter (101) and the light receiver (102) may be integrated together. This enables downsizing of the ultraviolet ray radiation apparatus (100). In addition, it is easier to determine the positional relationship between the light emitter (101) and the light receiver (102), the specifications of peripheral members, and the like. Thus, desired functions can be more reliably achieved.

### <<Other Embodiments>>

The above-described embodiment (including variations, where the same also applies hereinafter) illustrates the situation where the ultraviolet ray radiation apparatus (100) is provided in the indoor unit of the air conditioner (see FIG. 1). However, an application for which the ultraviolet ray radiation apparatus (100) is used is not specifically limited. The ultraviolet ray radiation apparatus (100) can be used for various applications such as UV light sources for an air cleaner, a sterilizer sterilizing a space such as a surgical operation room, a printer using UV-cure ink, and the like.

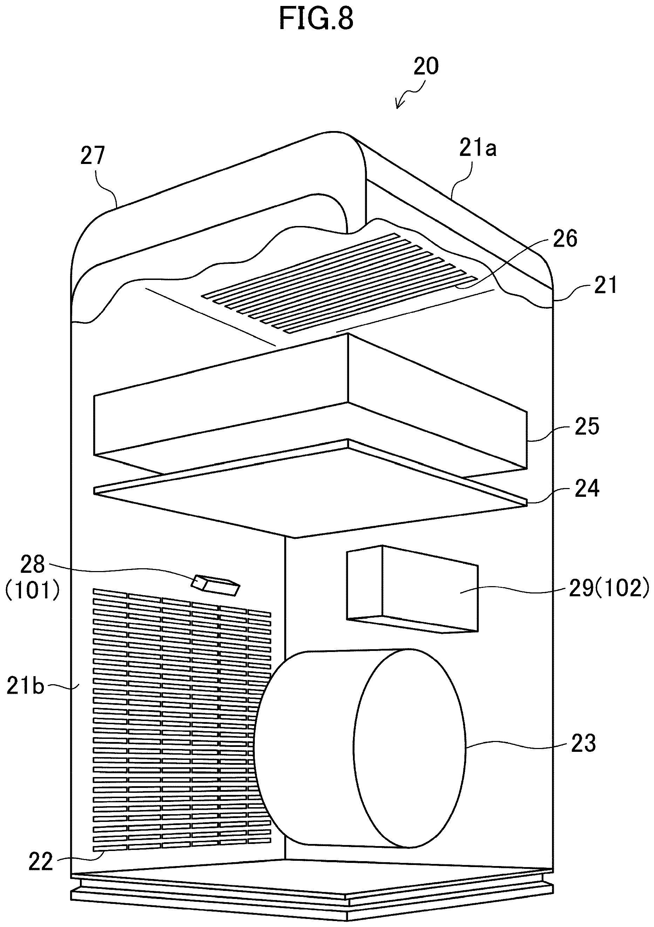

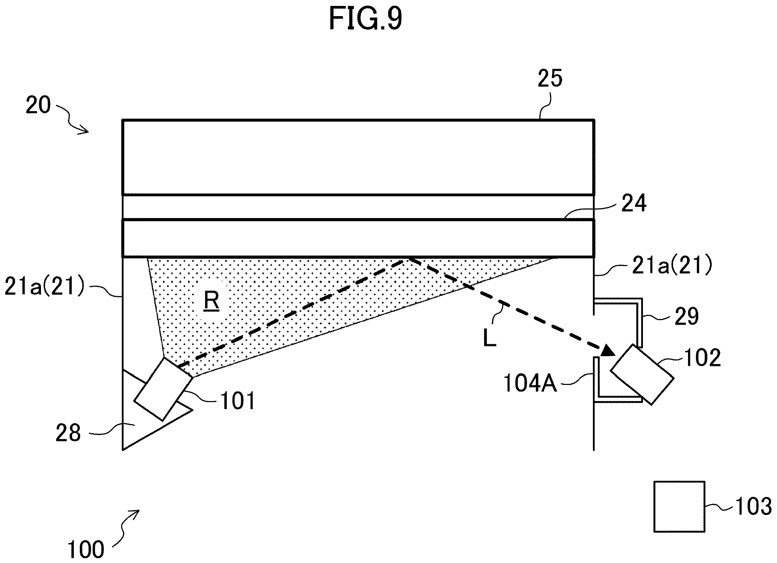

For example, as illustrated in FIGS. 8 and 9, the ultraviolet ray radiation apparatus (100) may be provided in an air cleaner (20). In FIGS. 8 and 9, the same reference characters are used to designate the same elements as those of the ultraviolet ray radiation apparatus (100) illustrated in FIG. 2. In FIG. 8, a part of a casing (21) is cut away so that the internal configuration of the air cleaner (20) is visible.

In the air cleaner (20), a fan (23), a filter (24), and a dust collection unit (25) are successively arranged on a ventilation path from the inlet (22) provided in a lower portion of a side surface (21b) of the substantially rectangular parallelepiped casing (21) to the outlet (26) provided in a top panel surface (21a) of the casing (21). An operating section (27) of the air cleaner (20) is arranged at the upper end of the side surface (21b) of the casing (21). When the fan (23) is operated in the air cleaner (20), indoor air is taken into the casing (21) through the inlet (22). Then, when the indoor air passes through the filter (24) and the dust collection unit (25), dust and the like are removed. Then, the resultant clean air is blown out of the casing (21) through the outlet (26).

The casing (21) includes the side surfaces (21b) facing each other, each of which is provided with a holder (28) holding the light emitter (101) and a holder (29) holding the light receiver (102). The light emitter (101) is arranged so as to be able to irradiate nearly the entire surface of the filter (24) with ultraviolet light. Irradiation with ultraviolet light enables sterilization and disinfection of the filter (24). The light receiver (102) is arranged such that ultraviolet light having reflected off the filter (24) enters the light receiver (102). The holder (29) holding the light receiver (102) includes a light shield (104A) as a protector that prevents ultraviolet light from entering the light receiver (102) directly from the light emitter (101). The control unit (103) of the ultraviolet ray radiation apparatus (100) may be configured as a part of a control unit of the air cleaner (20).

In the above-described embodiment, the protectors (104A, 104B, 104C) protecting the light receiver (102) from ultraviolet light is provided. Alternatively, the light receiver (102) may be arranged without the protectors (104A, 104B, 104C) in a region that is irradiated with ultraviolet light at an intensity less than or equal to a predetermined intensity so that the light receiver (102) is less deteriorated.

While the embodiments have been described above, it will be understood that various changes in form and details can be made without departing from the spirit and scope of the claims. The above embodiment may be combined or replaced as appropriate as long as the functions of the target of the present disclosure are not impaired.

### INDUSTRIAL APPLICABILITY

As can be seen from the foregoing description, the present disclosure is useful for an ultraviolet ray radiation apparatus.

### DESCRIPTION OF REFERENCE CHARACTERS

- 100: Ultraviolet Ray Irradiation Apparatus
- 101: Light Emitter (LED)
- 102: Light Receiver
- 103: Control Unit
- 104A: Protector (Light Shield)
- 104B: Protector (Reflector)
- 104C: Protector (Light Shield)
- 105: Optical Filter

## Claims

1. An ultraviolet ray irradiation apparatus comprising:
a light emitter (101) configured to emit ultraviolet light;
a light receiver (102) configured to output a signal varying with an amount of light received; and
a protector (104A, 104B, 104C) configured to protect the light receiver (102) from ultraviolet light.

2. The ultraviolet ray radiation apparatus of claim 1, wherein
the protector (104A, 104B, 104C) attenuates an amount of ultraviolet light entering the light receiver (102).

3. The ultraviolet ray radiation apparatus of claim 1 or 2, wherein
the protector (104A, 104B, 104C) includes a reflector (104B) configured to reflect ultraviolet light at least once until the ultraviolet light enters the light receiver (102) from the light emitter (101).

4. The ultraviolet ray radiation apparatus of any one of claims 1 to 3, wherein
the protector (104A, 104B, 104C) includes a light shield (104C) configured to block light with a wavelength shorter than a peak wavelength of ultraviolet light emitted from the light emitter (101).

5. The ultraviolet ray radiation apparatus of any one of claims 1 to 4, wherein
the protector (104A, 104B, 104C) includes a light shield (104C) configured to block light with a wavelength less than or equal to 280 nm.

6. The ultraviolet ray radiation apparatus of any one of claims 1 to 5, wherein
the light emitter (101) is an LED (101).

7. The ultraviolet ray radiation apparatus of claim 6 further comprising:
a control unit (103) configured to adjust a driving current of the LED (101) based on a signal from the light receiver (102) to control an amount of light emitted from the LED (101).

8. The ultraviolet ray radiation apparatus of any one of claims 1 to 7, wherein
an optical filter (105) configured to block light with a wavelength greater than or equal to 300 nm is provided in front of the light receiver (102).

9. The ultraviolet ray radiation apparatus of any one of claims 1 to 8, wherein the light emitter (101) and the light receiver (102) are integrated together.
